Europäisches Patentamt

European Patent Office

Office européen des brevets

(11) Veröffentlichungsnummer: **0 496 230 A1**

# EUROPÄISCHE PATENTANMELDUNG

(21) Anmeldenummer: **92100397.6**

(51) Int. Cl.5: **A61K 35/78**

(22) Anmeldetag: **11.01.92**

(30) Priorität: **23.01.91 DE 4101826**

(43) Veröffentlichungstag der Anmeldung:
**29.07.92 Patentblatt 92/31**

(84) Benannte Vertragsstaaten:
**AT BE CH DE DK ES FR GB GR IT LI LU NL PT SE**

(71) Anmelder: **ASTA Medica Aktiengesellschaft**
**Weismüllerstrasse 45**
**W-6000 Frankturt am Main 1(DE)**

(72) Erfinder: **Carle, Reinhold, Dr.**
**Im Taubhaus 56 A**
**W-6074 Rödermark(DE)**
Erfinder: **Gehringer, Claus, Dr.**
**Dahlmannstrasse 22**
**W-6000 Frankturt 60(DE)**
Erfinder: **Beyer, Jürgen, Dr.**
**Leuschnerstrasse 5**
**W-6700 Ludwigshafen(DE)**
Erfinder: **Engel, Jürgen, Prof.**
**Erlenweg 3**
**W-8755 Alzenau(DE)**

(54) **Herstellung eines Kamillenextraktes mit antimikrobiellen Eigenschaften.**

(57) Verfahren zur Herstellung eines polaren, antimikrobiell wirksamen Kamillenextraktes durch Extraktion von Kamillenzungenblüten mit üblichen Kamillenextraktionsmitteln und gegebenenfalls anschließender Sprühtrocknung.

EP 0 496 230 A1

Bei der Erfindung handelt es sich um einen hydrophilen Extrakt aus vorwiegend Kamillenzungenblüten, der beispielsweise für flüssige Vaginalpräparate verwendet werden kann. Für die Verwendung als Vaginaldusche können zusätzlich hierfür übliche bekannte Stoffe zugesetzt beziehungsweise mitverwendet werden.

Vaginalduschen sind flüssige Präparate zur Spülung der Vagina über einen unbestimmten Zeitraum zur Erzielung einer reinigenden, beruhigenden und erfrischenden Wirkung, zur Geruchsbeseitigung, zur Behebung geringfügiger Reizungen, zur Herabsetzung der Anzahl pathogener Mikroorganismen, zur Veränderung des pH und damit zur Anregung des Wachstums einer normalen Vaginalflora, zur Erzielung einer adstringierenden Wirkung, Reduzierung der Oberflächenspannung zur Erzeugung einer mukolytischen oder proteolytischen Wirkung. Vaginalduschen werden häufig zur intravaginalen Kontrazeption benutzt. Es ist außerdem vorgeschlagen worden, sie als Methode zur Beeinflussung des Geschlechts der Nachkommen zu verwenden, indem vor dem Koitus saure oder basische Duschen benutzt werden (SHETTLES, L.B. (1970): Intern. J. Gynaecol. Obstetrics 8, 643).

Die antiseptischen Eigenschaften des pflanzenhaltigen ätherischen Öls werden im allgemeinen auf die enthaltenen flüchtigen Verbindungen zurückgeführt (TYLER, V.E. et al.: Pharmacognosy, 7. Aufl., Lea-Febiger, Philadelphia 1976, S. 137). In niedrigen Konzentrationen übt Kamillenöl eine bakteriostatische und bakterizide Wirkung auf grampositive Pathogene aus und zeichnet sich durch eine bemerkenswerte fungizide Wirkung gegen Candida albicans aus (AGGAG, E. und T. YOUSEF (1972): Planta Med. 22, 140). In-vivo-Tests zum Vergleich der lipophilen Bestandteile der Kamillenblüten haben ergeben, daß (-)-α-Bisabolol die stärkste bakteriostatische Wirkung besaß (SZABO-SZALONTAI et al. (1975): Pharm. Ztg. 120, 982; Dtsch. Apoth. Ztg. 115, 912).

Über eine aus Kamillenblüten gewonnene apolare Fraktion wurde berichtet, daß sie auf das Wachstum von aus dem Vaginalsekret isolierten Mikroorganismen eine Hemmwirkung ausübt. Hierbei wurden die Kamillenblüten mit Ethanol unter Rückfluß extrahiert, der Extrakt wurde filtriert und das Lösungsmittel wurde verdampft. Anschließend wurde der pastöse Rückstand mehrmals mit kaltem Hexan durchgearbeitet. Der Rückstand wurde in heißem Methanol gelöst, filtriert und die Lösung wurde zur Trockne eingedampft. Dieser Extrakt enthält keine Flavone und zeigt keine bakteriostatische Wirkung (TURBARO, A. et al.; Europ. Pat. App. Nr. 0096016 vom 01.06.1983).

Die Zungenblütchen der Kamille enthalten hauptsächlich Flavone, zum Beispiel Apigenin-7-Glucosid, Apigenin-7-Acetylglucosid und Apigenin und andere polare Verbindungen. Flüchtige Bestandteile (= ätherisches Öl) der Kamille finden sich insbesondere in den Röhrenblüten des Blütenköpfchens, in den Zungenblüten sind dagegen nur geringe Mengen nachweisbar.

Es wurde nun überraschend gefunden, daß eine aus Kamillezungenblüten durch Extraktion mit heißem Wasser gewonnene Fraktion eine beachtliche bakteriostatische und trichomonazide Wirkung aufweist. Diese Fraktion hat völlig unerwartet eine bakteriostatische Wirkung gezeigt, obwohl sie ausschließlich aus polaren Verbindungen, zum Beispiel Flavonen, Flavonglycosiden und Polysacchariden besteht.

Die bakteriostatische Wirkung der aus dieser Kamillenfraktion und aus Kombinationen mit anderen wirksamen Substanzen bestehenden Präparate wurde mit Hilfe der Standardmethode der Serienverdünnung in einer Flüssigkeit und anschließender Ermittlung der Zahl lebensfähiger Organismen auf einem festen Nährboden bestimmt (MEINGASSNER et al. (1981): Arzneim. Forsch. 31, 6). Für die antibakteriellen Tests wurden Isolate vaginaler Mikroorganismen benutzt.

Die Bestimmung erfolgte zum Beispiel in folgender Weise:
Ein Isolat vaginaler Mikroorganismen wurde auf ein Agar-Nährmedium übertragen, 24 Stunden bei 37°C kultiviert und mit sterilem Wasser auf ein flüssiges Medium übergeführt. Das als Inoculum verwendete flüssige Kulturmedium enthielt $10^4$ Mikroorganismen per ml und wurde in 10 ml-Portionen auf 200 ml Flaschen verteilt. Diesen Proben wurde der Kamillenextrakt in verschiedenen Konzentrationen zugesetzt (1 bis 10 mg/ml). Nach 24-stündiger Inkubation bei 37°C wurde die Anzahl der überlebenden Mikroorganismen bestimmt.
Das Wachstum eines Isolats vagnialer Mikroorganismen wurde mit 5 mg/ml zu 100 % gehemmt (5.03.1990).

Die Erfindung betrifft einen Extrakt aus Kamillenblüten mit bakteriostatischer und trichomonazider Wirkung, der erhältlich ist durch Extraktion von Kamillezungenblüten, wobei auch geringere Mengen Kamillenröhrenblüten mitverwendet werden können und in diesem Fall auf einen Gewichtsteil Zungenblüten bis maximal 0,3 Gewichtsteile Röhrenblüten eingesetzt werden können und ein Verfahren zu dessen Herstellung. Dieses Verfahren ist dadurch gekennzeichnet, daß man Kamillenzungenblüten, wobei auch geringere Mengen Kamillenröhrenblüten mitverwendet werden können und in diesem Fall auf einen Gewichtsteil Zungenblüten bis maximal 0,3 Gewichtsteile Röhrenblüten eingesetzt werden können, unter Verwendung von mit Wasser mischbaren organischen polaren Lösungsmitteln mit Siedepunkten zwischen 30 und 90 °C oder Mischungen solcher Mittel mit Wasser bei Tempe-

raturen zwischen 60 - 100°C, mindestens 1 Minute extrahiert und gegebenenfalls das Extraktionsmittel entfernt.

Weiterhin betrifft die Erfindung die Verwendung eines derartigen Extraktes als Vaginaldusche, sowie entsprechende Zubereitungen.

Insbesondere handelt es sich bei einer solchen Zubereitung um eine schwach gelbliche wäßrige Lösung mit einem pH-Wert zwischen 3,0 und 4,0, einem spezifischen Gewicht von 1,0004 - 1,0006 enthaltend die polaren Kamilleninhaltsstoffe Apigenin, Apigenin-7-Glucosid und Apigenin-7-Acetylglucosid mit einem Gesamtflavonoidgehalt zwischen 1 und 30 mg/ml, 0,1 - 3,0 Gewichts% eines physiologisch verträglichen Puffers zur Einstellung des pH-Wertes zwischen 3,0 und 4,0, sowie gegebenenfalls weitere physiologisch verträgliche Hilfs-, Konservierungs-, Duft-, Aromastoffe und/oder Farbstoffe.

Die Herstellung einer derartigen Vaginalduschenformulierung erfolgt zum Beispiel unter Verwendung eines erfindungsgemäßen Extrakts, indem man den Extrakt gegebenenfalls mit oder ohne die hierfür üblichen Hilfsstoffe sprühtrocknet, das erhaltene Produkt in Wasser auflöst, mit physiologisch unbedenklichen pharmazeutischen Hilfs- und Zusatzstoffen versetzt filtriert, sterilisiert und unter aseptischen Bedingungen abfüllt.

Zur Herstellung des erfindungsgemäßen Kamillenextraktes

Wesentliches Ausgangsmaterial sind Zungenblüten der Kamille, wobei es sich um frische oder getrocknete Blüten handeln kann. Im Falle von getrockneten Blüten handelt es sich stets um Blüten, die bei 20 bis 60 °C bis zur Gewichtskonstanz getrocknet wurden (Trocknung erfolgt beispielsweise bei 40 °C). Getrocknete Blüten enthalten maximal bis zu 25 % Gewichts% Wasser.

Bei der Verwendung von frischem Material muß die Menge des Ausgangsmaterials fünfmal größer sein als bei getrocknetem Material.

Bei der Extraktion der Zungenblüten können gegebenenfalls auch geringere Mengen an Kamilleröhrenblüten mitverwendet werden.

Anstelle von getrockneten Blüten kann jeweils auch das entsprechende Drogenäquivalent an frischen Blüten (Zungenblüten und/oder Röhrenblüten) eingesetzt werden.

Im Falle von Mischungen aus Zungenblüten und Röhrenblüten setzen sich diese wie folgt zusammen:

Es können maximal bis zu 0,3 Gewichtsteile getrocknete Kamilleröhrenblüten pro 1 Gewichtsteil getrocknete Kamillezungenblüten zugesetzt werden (also 0 - 0,3 Gewichtsteile Kamilleröhrenblüten).

Falls frische Kamillezungenblüten eingesetzt werden, gilt hinsichtlich des Anteils an frischen Röhrenblüten ebenfalls das Verhältnis 0 - 0,3 Gewichtsteile frische Röhrenblüten auf einen Gewichtsteil frische Zungenblüten.

Bei Verwendung von frischen Kamillezungenblüten und getrockneten Kamillenröhrenblüten werden vorzugsweise auf einen Gewichtsteil frische Kamillezungenblüten 0 - 0,1 Gewichtsteile getrocknete Kamilleröhrenblüten (Trocknung stets wie angegeben) verwendet.

Bei Verwendung von getrockneten Kamillezungenblüten und frischen Kamillenröhrenblüten werden vorzugsweise auf einen Gewichtsteil getrocknete Zungenblüten 0 -1,5 Gewichtsteile frische Kamilleröhrenblüten verwendet.

Als Extraktionsmittel kommen beispielsweise flüssige Mittel in Frage mit Siedepunkten zwischen 50 und 150 °C, vorzugsweise 56 und 120 °C, insbesondere 56 und 105 °C. Beispiele für solche Mittel sind: Wasser, primäre, sekundäre oder tertiäre ein- oder mehrwertige gesättigte aliphatische Alkohole, wie zum Beispiel Methanol, Ethanol, Propanol, Isopropanol, aliphatische gesättigte Ketone mit 1 - 4 C-Atomen (Aceton, Methylethylketon) oder Mischungen dieser Mittel.

Die Extraktionstemperatur liegt beispielsweise zwischen 60 bis 100°C, vorzugsweise 70 bis 100 °C, insbesondere 90 bis 100 °C.

Die Extraktion erfolgt in den für Kamillenextraktionen üblichen Apparaten beziehungsweise Vorrichtungen. Beispielsweise kommen in Frage:

Auf einen Gewichtsteil frische Zungenblüten werden beispielsweise 2 bis 10, vorzugsweise 4 bis 8, insbesondere 4 bis 6 Gewichtsteile des beziehungsweise der zuvor genannten Extraktionsmittel verwendet.

Auf einen Gewichtsteil trockene Zungenblüten werden 10 bis 60, vorzugsweise 20 bis 50, insbesondere 20 bis 40 Gewichtsteile des beziehungsweise der zuvor genannten Extraktionsmittel verwendet.

Diese Mengen an Extraktionsmitteln gelten auch, wenn außer den Zungenblüten noch Röhrenblüten mitverwendet werden und zwar unabhängig davon, ob es sich bei den Röhrenblüten um getrocknete oder frische Blüten handelt.

Die Extraktionszeit beträgt beispielsweise 1 bis 15, vorzugsweise 3 bis 10 insbesondere 3 bis 5 Minuten.

Nach der Extraktion durchläuft das extrahierte Material beispielsweise eine Presse, wird filtriert und gegebenenfalls unter Verwendung von üblichen Hilfs- und Zusatzstoffen wie zum Beispiel Lactose und Zellulosederivaten sprühgetrocknet.

Bei der Sprühtrocknung können beispielsweise auch andere hierfür übliche Stoffe verwendet werden.

Der so erhaltene Trockenextrakt ist dann zur Herstellung von therapeutischen Zusammensetzungen (zum Beispiel für eine Vaginaldusche) geeignet. Die Löslichkeit des so erhaltenen sprühgetrockneten Extraktes ist in Wasser am besten (1 Teil des Extraktes löst sich beispielsweise in ca. 5 Teilen Wasser) und nimmt mit der geringer werdenden Polarität des Lösungsmittels ab.

Weitere Lösungsmittel für den Extrakt sind physiologisch verträgliche Lösungsmittel polaren Charakters, wie zum Beispiel Alkohole mit Kettenlängen bis 6 C-Atomen.

Die therapeutischen Zusammensetzungen (Vaginaldusche zum Beispiel) werden beispielsweise durch Auflösen des sprühgetrockneten Extrakts in gereinigtem Wasser erhalten, wobei mittels Milchsäure/Natriumlactat ein pH-Wert von 3,0 bis 4,0 eingestellt wird (zum Beispiel durch ein Gemisch aus Milchsäure und Natriumlactat im Verhältnis 2 zu 1). Die Konzentration der Milchsäure/Natriumlactat Mischung beträgt hierbei beispielsweise 0,1 bis 3,0 Gewichts%, vorzugsweise 0,4 bis 1,3 Gewichts%.

Weitere übliche Puffer für Einstellung eines pH-Wertes von 3,0 bis 4,0 kommen ebenfalls in Frage.

Den therapeutischen Zusammensetzungen können gegebenenfalls noch weitere für Vaginaluschen übliche Zusatzstoffe beziehungsweise Hilfsstoffe zugesetzt werden.

Als derartige Zusatzstoffe kommen beispielsweise folgende Stoffe in Frage:

(Die folgenden angegebenen Gewichtsprozente beziehen sich jeweils auf die fertige Endzusammensetzung).

Alkali- oder Erdalkalimetallsalze der Propionsäure, wie zum Beispiel Calcium- oder Natriumpropionat, beispielsweise in Konzentrationen bis zu 20 Gewichts%, Alkalimetallsalze der Sorbinsäure, wie zum Beispiel Kaliumsorbat (beispielsweis in Konzentrationen zwischen 1 und 3 %), Polyvinylpyrrolidon-Iodid (Konzentrationen beispielsweise zwischen 0,1 und 0,3 Gewichts%), Natriumbenzoat, Benzoesäure, Natriumcitrat.

Vorzugsweise handelt es sich hierbei um physiologisch verträgliche Stoffe, die Reizungen der Schleimhaut günstig beeinflussen.

Weiterhin kommen oberflächenaktive Substanzen wie zum Beispiel anionische und nichtionische Detergentien in Frage. Als anionische Detergentien können zum Beispiel Dioctyl-Natrium-sulfosuccinat oder Natriumlaurylsulfat (Konzentrationen zwischen 0,002 und 0,02 Gewichts%) verwendet werden; Dioctyl-Natrium-sulfosuccinat bevorzugt in einer Menge von 0,002 bis 0,01 Gewichts%; Natriumlaurylsulfat bevorzugt in einer Menge von 0,01 - 0,02 Gewichts%.

Als nichtionische Detergentien kommen beispielsweise Octoxynol-9 und/oder Nonoxynol-9 (Konzentrationen zwischen 0,01 Gewichts% und 0,1 Gewichts% in Frage. Octoxynol-9 vorzugsweise in der Konzentration von 0,08 bis 0,09 Gewichts%; Nonoxynol-9 vorzugsweise in der Konzentration 0,01 bis 0,02 Gewichts%.

Zur Änderung des vaginalen pH-Wertes und zur Förderung des normalen Wachstums der Vaginalflora, kann Essigsäure zwischen 4 und 6 Gewichts% eingesetzt werden, desweiteren Borverbindungen wie Borsäure, Natriumborat, Natriumperborat und Borglycerin in Konzentrationen zwischen 0 und 1,0 Gewichts%, Zitronensäure zwischen 0,1 und 0,5 %, Milchsäure oder Alkalimetallsalze der Milchsäure, insbesondere Natriumlactat oder Gemische aus Natriumlactat und Milchsäure mit einem Gesamtgehalt von 0,4 - 3,0 Gewichts%, Natriumbicarbonat, Natriumkarbonat und/oder Weinsäure (Konzentration jeweils von 0,02 bis 3,0 Gewichts%).

Als adstringierende Mittel können die erfindungsgemäßen pharmazeutischen Zubereitungen (Vaginaldusche) beispielsweise Aluminiumverbindungen wie zum Beispiel Kaliumaluminiumsulfat, Ammoniumaluminiumsulfat (0,03 und 0,06 Gewichts%), Borverbindungen wie zum Beispiel Borsäure, Natriumborat, Natriumperborat und Borglycerin, Zinksulfat (jeweils 0,001 bis 0,02 Gewichts%) enthalten.

Darüberhinaus können die erfindungsgemäßen pharmazeutischen Zubereitungen mukolytische und proteolytische Substanzen enthalten, wie zum Beispiel: Alkylarylsulfonate (Konzentrationen bis zu 0,1 Gewichts%), Milchsäure, Alkalilactate, Papain (Konzentration jeweils zwischen 0,005 bis 0,1 Gewichts%).

Vorzugsweise kommen als solche Hilfs- und Zusatzstoffe in Frage:
Octoxynol-9 (Gehalt zum Beispiel 0,08 bis 0,1 Gewichts%) und/ oder Nonoxynol-9 (Gehalt zum Beispiel 0,01 Gewichts%) sowie Natriumbenzoat (Gehalt zum Beispiel 0,1 bis 0,5 Gewichts%).

Die zuvor erwähnten Zusatzstoffe können einzeln oder auch in Kombination verwendet werden.

Als weitere Zusatzstoffe kommen beispielsweise in Frage: Duft- beziehungsweise Aromastoffe und Farbstoffe, die in den jeweiligen stoffspezifisch zulässigen Konzentrationen eingesetzt werden. Bei den Duftstoffen liegt die Konzentration in der Regel unter 1 Gewichts%.

Die so erhaltene Zusammensetzung (Vaginaldusche) wird dann beispielsweise durch ein Filter der Porenweite 0,1 bis 2 $\mu$m, vorzugsweise 0,22 $\mu$m filtriert, bei 120 °C 20 Minuten mit Wasserdampf sterilisiert und unter sterilen Bedingungen in die Endverpackungen abgefüllt.

Beispiel 1:

Eine Mischung aus 3,750 kg Zungenblütchen und 0,425 kg Röhrenblütchen wird mit 125 kg Wasser 10 Minuten lang bei 90°C extrahiert. Der

filtrierte Extrakt lieferte 0,75 kg des sprühgetrockneten Produkts. Nach Zusatz von 873,00 kg gereinigtem Wasser werden in der so erhaltenen wässrigen Mischung 3,50 kg Milchsäure 90 % und 4,63 kg Natriumlactat 50 % gelöst. Die Mischung wird nun filtriert, sterilisiert und unter aseptischen Bedingungen abgefüllt.

Beispiel 2:

Eine Mischung aus 3,750 kg Zungenblütchen und 0,425 kg Röhrenblütchen wird mit 125 kg Wasser 10 Minuten lang bei 90°C extrahiert. Der filtrierte Extrakt lieferte 0,75 kg des sprühgetrockneten Produkts. Nach Zusatz von 873,00 kg gereinigtem Wasser werden in der so erhaltenen wässrigen Mischung 3,50 kg Milchsäure 90 % und 2,10 kg Natriumlactat 50 %, 0,77 kg Octoxynol-9 und 1,31 kg Natriumbenzoat gelöst. Die Mischung wird nun filtriert, sterilisiert und in die Endverpackung abgefüllt.

Beispiel 3:

Eine Mischung aus 3,550 kg Zungenblütchen und 0,625 kg Röhrenblütchen wird mit 125 kg Wasser 15 Minuten lang bei 95°C extrahiert. Der filtrierte Extrakt lieferte 0,80 kg des sprühgetrockneten Produkts. Nach Zusatz von 931,00 kg gereinigtem Wasser werden in der so erhaltenen wässrigen Mischung 3,73 kg Milchsäure 90 % und 2,24 kg Natriumlactat 50 %, 0,82 kg Octoxynol-9 und 1,40 kg Natriumbenzoat gelöst. Die Mischung wird dann filtriert, sterilisiert und in die Endverpackungen abgefüllt.

**Patentansprüche**

1. Extrakt aus Kamillenblüten mit bakteriostatischer und trichomonazider Wirkung, erhältlich durch Extraktion von Kamillezungenblüten, wobei auch geringere Mengen Kamillenröhrenblüten mitverwendet werden können und in diesem Fall auf einen Gewichtsteil Zungenblüten bis maximal 0,3 Gewichtsteile Röhrenblüten eingesetzt werden können.

2. Schwach gelbliche wäßrige Lösung mit einem pH-Wert zwischen 3,0 und 4,0, einem spezifischen Gewicht von 1,0004 - 1,0006 enthaltend die polaren Kamilleninhaltsstoffe Apigenin, Apigenin-7-Glucosid und Apigenin-7-Acetylglucosid mit einem Gesamtflavonoidgehalt zwischen 1 und 30 mg/ml, 0,1 - 3,0 Gewichts% eines physiologisch verträglichen Puffers zur Einstellung des pH-Wertes zwischen 3,0 und 4,0, sowie gegebenenfalls weitere physiologisch verträgliche Hilfs-, Konservierungs-, Duft-, Aromastoffe und/oder Farbstoffe.

3. Verwendung einer Zusammensetzung nach Anspruch 1 oder 2 als Vaginaldusche.

4. Verfahren zur Herstellung eines Kamillen-Extrakts nach Anspruch 1, dadurch gekennzeichnet, daß man Kamillenzungenblüten, wobei auch geringere Mengen Kamillenröhrenblüten mitverwendet werden können und in diesem Fall auf einen Gewichtsteil Zungenblüten bis maximal 0,3 Gewichtsteile Röhrenblüten eingesetzt werden können, unter Verwendung von mit Wasser mischbaren organischen polaren Lösungsmitteln oder Mischungen solcher Mittel mit Wasser bei Temperaturen zwischen 60 -100°C, mindestens 1 Minute extrahiert und gegebenenfalls das Extraktionsmittel entfernt.

5. Herstellung einer Vaginalduschenformulierung unter Verwendung eines Extrakts nach Anspruch 1 oder Anspruch 4 gewonnenen Extrakts, dadurch gekennzeichnet, daß man den Extrakt gegebenenfalls mit oder ohne die hierfür üblichen Hilfsstoffe sprühtrocknet, das erhaltene Produkt in Wasser auflöst, mit physiologisch unbedenklichen pharmazeutischen Hilfs- und Zusatzstoffen versetzt filtriert, sterilisiert und unter aseptischen Bedingungen abfüllt.

Europäisches
Patentamt

# EUROPÄISCHER RECHERCHENBERICHT

Nummer der Anmeldung

| EINSCHLÄGIGE DOKUMENTE | | | EP 92100397.6 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| X | EP - A - 0 330 240 (ROBUGEN GMBH PHARMAZEUTISCHE FABRIK) * Seite 3, Zeile 24 - Seite 4, Zeile 21 * -- | 1,2 | A 61 K 35/78 |
| A | EP - A - 0 154 872 (DEGUSSA AKTIENGESELLSCHAFT) * Ansprüche * ---- | 1,4 | |

| | | | RECHERCHIERTE SACHGEBIETE (Int. Cl.⁵) |
|---|---|---|---|
| | | | A 61 K |

Der vorliegende Recherchenbericht wurde für alle Patentansprüche erstellt.

| Recherchenort | Abschlußdatum der Recherche | Prüfer |
|---|---|---|
| WIEN | 23-04-1992 | SCHNASS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82